# EUROPEAN PATENT SPECIFICATION

(11) **EP 4 340 959 B1**
(45) Date of publication and mention of the grant of the patent: **11.11.2026**
(21) Application number: 22803477.3
(22) Date of filing: 18.05.2022
(51) Int. Cl.: A63B 21/055, A63B 21/06, A63B 24/00, A63B 71/06, G06F 17/18, A61B 5/11, A63B 21/068

(54) **A SENSOR-ENABLED PLATFORM CONFIGURED TO MEASURE ATHLETIC ACTIVITY**
SENSORAKTIVIERTE PLATTFORM MIT KONFIGURATION ZUR MESSUNG VON SPORTLICHER AKTIVITÄT
PLATEFORME ACTIVÉE PAR CAPTEUR, CONÇUE POUR MESURER L'ACTIVITÉ ATHLÉTIQUE

(30) Priority: 18.05.2021 AU 2021901479; 25.08.2021 AU 2021221661; 09.12.2021 AU 2021903980; 10.03.2022 AU 2022900585
(43) Date of publication of application: 27.03.2024
(73) Proprietor: Hit Tekk Pty Ltd, Queenscliff, NSW 2096 (AU)
(72) Inventor: GRAHAM, Mat, Queenscliff, NSW 2096 (AU); WARD, Matthew, Queenscliff, NSW 2096 (AU)
(74) Representative: Haseltine Lake Kempner LLP
(86) International application number: PCT/AU2022/050480
(87) International publication number: WO 2022/241516

(56) References cited:
- CN-A- 110 613 915
- KR-A- 20210 037 775
- KR-A- 20210 108 173
- KR-B1- 102 032 681
- US-A1- 2011 218 077
- US-A1- 2018 214 729
- US-A1- 2018 369 640
- US-A1- 2019 344 123
- US-A1- 2020 023 229
- US-A1- 2020 047 055
- US-A1- 2020 205 720
- US-A1- 2021 060 377
- FORCEDECKS SPORTS PERFORMANCE - FORCEDECKS, GAME-CHANGING TECHNOLOGY, 19 July 2016 (2016-07-19), XP093009238, Retrieved from the Internet <URL:https://www.youtube.com/watch?v=Yt59eEnsybY>

## Description

### FIELD OF THE DISCLOSURE

The present disclosure relates, in various embodiments, to a sensor-enabled platform configured to measure athletic activity. For example, some embodiments provide a platform which is configured to enable functional workouts, including medicine ball workouts, cardiovascular workouts, and/or resistance training, with the sensors being configured to measure participant performance in performing associated exercises. Although the present disclosure is described primarily in connection with such examples, it will be appreciated that further embodiments find wider application.

### BACKGROUND

Any discussion of the background art throughout the specification should in no way be considered as an admission that such art is widely known or forms part of common general knowledge in the field.

A wide range of electronic devices which measure athletic activity are known. Common examples include cardiovascular training apparatus, such as treadmills, rowing machines, and stationary bicycles. With an increase in the popularity of functional training, for example via Cross Fit and other regimes, there have been various efforts made to include performance sensors in a wider range of equipment, thereby to measure/quantify performance in further athletic activities. One example is the incorporation of accelerometers into medicine balls, thereby to measure attributes of ball motion.

It is an object of the present invention to overcome or ameliorate at least one of the disadvantages of the prior art, or to provide a useful alternative.
Patent document US2011/218077A1 discloses an effort-measuring apparatus for use with a balance-and-weight sensing platform, including anchor points for resistance mechanisms.

### SUMMARY OF THE INVENTION

The present invention is defined by the appended claims.

### OUTLINE OF THE DISCLOSURE

The following passages describe aspects and examples useful for understanding the detailed embodiments. The invention for which protection is sought is defined by the appended claims.

One embodiment provides a device configured to measure athletic activity, the device including:
a platform having an upper surface configured to receive an applied force;
one or more sensors configured to monitor the force applied to the upper surface of the platform;
a processing unit configured to process data received from the one or more sensors, thereby to derive metrics representative of athletic performance associated with a predefined category of exercise which applies the force to the upper surface.

One embodiment provides a device wherein the one or more sensors configured to monitor force applied to the upper surface of the platform include a sensor configured to measure a metric associated with a force of an object impacting the upper surface.

One embodiment provides a device wherein the one or more sensors configured to monitor force applied to the upper surface of the platform include a sensor configured to measure a metric associated with a force of a body weight exercise being performed by a user standing or lying on the upper surface.

One embodiment provides a device wherein the one or more sensors configured to monitor force applied to the upper surface of the platform include a sensor configured to measure a metric associated with a force applied to the platform via a combination of body weight and tension in a tether configured to provide resistive force.

One embodiment provides a device wherein the device includes a base, and wherein the tether is coupled to the base such that when the user increases tension in the tether whilst standing or lying on the platform, that increases the applied force.

One embodiment provides a device the metrics representative of athletic performance include a measure representative of cumulative force over a specified time period.

One embodiment provides a device wherein the metrics representative of athletic performance include a measure representative of average impact force over a specified time period.

One embodiment provides a device wherein the predefined category of exercise is an exercise performed repetitively as repetitions, and the metrics representative of athletic performance are calculated for each repetition and for a collection of repetitions.

One embodiment provides a device wherein the sensors record the applied force as time domain information.

One embodiment provides a device wherein the metrics are derived from the time domain information.

One embodiment provides a device wherein a subset of the metrics are derived from first and/or second order derivatives of the time domain information.

One embodiment provides a device 11 wherein the predefined category of exercise is an exercise performed repetitively as repetitions, and wherein the time domain information is processed thereby to identify and assess individual repetitions.

One embodiment provides a device wherein the processing unit is configured to process a time domain signal thereby to determine: (i) a weight of a user; (ii) a weight of the object; and /or (iii) a combined weight of the user and the object.

One embodiment provides a device wherein the processing unit is configured to cause display of data derived from the one or more sensors on a display screen.

One embodiment provides a device wherein the device includes the display screen.

One embodiment provides a device wherein the display screen is a remote display screen, and wherein the device includes an interface for connection of the remote display screen.

One embodiment provides a device wherein the processing unit is configured to execute software instructions representative of one or more predefined workout programs, thereby to, via a display screen: (i) instruct a user to perform athletic activities; and (ii) display data derived from the one or more sensors thereby to provide feedback data regarding athletic performance in an executing one or the one or more predefined workout programs.

One embodiment provides a device wherein the feedback data includes data representative of one or more of the following:
- Applied force;
- Time under tension/force
- Cumulative applied force during a workout period;
- Average per-event force during a workout period;
- Effort, derived from force normalised for object mass;
- Repetition rate;
- Average effort;
- Object acceleration;
- Lift force metrics;
- Acceleration;
- Total mass lifted;
- Calories burned; and
- A performance metric based on object mass and user mass.

One embodiment provides a device wherein the device includes a multi-user interface configured to enable display of relative operation of two or more of the platforms having a substantially planar upper surface and a respective one or more sensors configured to monitor force applied to the upper surface of the platform.

One embodiment provides a device wherein the multi user interface enables display of a display screen of competitive metrics for a plurality of simultaneous users.

One embodiment provides a device configured to measure athletic activity, the device including:
a platform having an upper surface configured to be impacted by a portable object;
one or more sensors configured to monitor force applied to the upper surface of the platform;
a processing unit configured to process data received from the one or more sensors, thereby to derive metrics representative of athletic performance associated with impacting a portable object onto the upper surface.

One embodiment provides a device wherein the one or more sensors configured to monitor force applied to the upper surface of the platform include a sensor configured to measure a metric associated with a force of the object impacting the upper surface.

One embodiment provides a device wherein the metrics representative of athletic performance associated with impacting a portable object onto the upper surface include a measure representative of cumulative impact force over a specified time period.

One embodiment provides a device wherein the metrics representative of athletic performance associated with impacting a portable object onto the upper surface include a measure representative of average impact force over a specified time period.

One embodiment provides a device wherein the one or more sensors configured to monitor force applied to the upper surface of the platform include: (i) a first set of one or more sensors configured to monitor force applied to a first region of the platform; and (ii) a second set of one or more sensors configured to monitor force applied to a second region of the platform.

One embodiment provides a device wherein the first region of the platform represents an impact target region onto which the object is impacted during normal use, and wherein the second region of the platform represents a standing region on which a user stands during normal use.

One embodiment provides a device wherein the one or more sensors configured to monitor force applied to the upper surface of the platform are configured to measure force applied both an impact target region of the platform onto which the object is impacted during normal use, and a standing region of the platform on which a user stands during normal use.

One embodiment provides a device wherein the processing unit is configured to process a time domain signal thereby to determine a weight of the object.

One embodiment provides a device wherein the processing unit is configured to process a time domain signal thereby to determine: (i) a weight of a user; (ii) a weight of the object; and /or (iii) a combined weight of the user and the object.

One embodiment provides a device wherein the processing unit is configured to cause display of data derived from the one or more sensors on a display screen.

One embodiment provides a device wherein the device includes the display screen.

One embodiment provides a device wherein the display screen is a remote display screen, and wherein the device includes an interface for connection of the remote display screen.

One embodiment provides a device wherein the processing unit is configured to execute software instructions representative of one or more predefined workout programs, thereby to, via a display screen: (i) instruct a user to perform athletic activities; and (ii) display data derived from the one or more sensors thereby to provide feedback data regarding athletic performance in an executing one or the one or more predefined workout programs.

One embodiment provides a device wherein the feedback data includes data representative of one or more of the following:
- Impact force;
- Time under tension/force
- Cumulative impact force during a workout period;
- Average impact force during a workout period;
- Effort, derived from impact force normalised for object mass;
- Repetition rate;
- Average effort;
- Object acceleration;
- Lift force metrics;
- Object acceleration;
- Total mass lifted;
- Calories burned; and
- A performance metric based on object mass and user mass.

One embodiment provides a device wherein the processing unit is configured to measure: (i) a mass of the portable object; (ii) an impact force at which the portable object strikes the platform; and (iii) based on (i) and (ii), derive a metric for effort associated with striking the object on the platform.

One embodiment provides a device wherein the portable object is an object configured to strike the platform with minimal rebound.

One embodiment provides a device wherein the portable object is a medicine ball.

One embodiment provides a device wherein the device includes a multi-user interface configured to enable display of relative operation of two or more of the platform having a substantially planar upper surface and a respective one or more sensors configured to monitor force applied to the upper surface of the platform.

One embodiment provides a device 39 wherein the multi user interface enables display of a display screen of competitive metrics for a plurality of simultaneous users.

One embodiment provides a system configured to enable multiple-activity functional workouts via distributed exercise machines, the system including:
a machine configuration database which contains a plurality of modality data sets, wherein each modality data set includes, for a specific exercise modality, data representative of:
   (i) device configuration data executable via a given one of the exercise machines thereby to configure that device to receive physical input representative of the specific exercise modality;
   (ii) processing configuration data executable via a that one of the exercise machines thereby to configure the device to process physical input representative of the specific exercise modality when operated in accordance with the device configuration data;
a playlist selector module, wherein the playlist selector module is configured to enable selection of a defined playlist, wherein each playlist is representative of instructions to deliver a plurality of exercise modalities in a predefined sequence;
a playlist delivery module, wherein the playlist delivery module is responsive to a selection via the playlist selector module thereby to cause delivery of data via a network to a defined one or more of the distributed exercises devices, thereby to configure those devices to enable execution of the selected playlist.

One embodiment provides a system wherein configuring the exercise machine to receive physical input representative of the specific exercise modality includes setting parameters for one or more sensor devices.

One embodiment provides a system wherein the parameters for the one or more sensor devices include sampling rates and/or sensitivity settings.

One embodiment provides a system wherein configuring the exercise machine to receive physical input representative of the specific exercise modality includes configuring the device to deliver predefined user directions.

One embodiment provides a system wherein the directions are provided via any one or more of: (i) a display; (ii) lights; (iii) communication via a user smartphone; (iv) audio instructions delivered by a speaker of the device; and (v) audio instructions delivered via a wireless headphone.

One embodiment provides a system wherein configuring the exercise machine to process physical input representative of the specific exercise modality includes setting parameters for determining any one or more of the following in respect of an exercise of the specific modality:
(i) that an exercise repetition has been performed in accordance with defined requirements;
(ii) a cumulative number of repetitions of the exercise that have been performed in accordance with defined requirements;
(iii) performance attributes associated with a repetition of the exercise performed in accordance with defined requirements;
(iv) cumulative performance attributes of a plurality of the repetitions of the exercise performed in accordance with defined requirements;
(v) timing attributes for one or more the repetitions of the exercise performed in accordance with defined requirements.

One embodiment provides a system wherein the device is configured to determine performance attributes associated with a repetition of the exercise performed in accordance with defined requirements, and those performance attributes include any one or more of the following:
(i) a metric representative of physical exertion;
(ii) a metric representative of technical form;
(iii) a metric representative of adherence to instruction.

One embodiment provides a system wherein each playlist is representative of instructions to deliver a plurality of exercise modalities: (i) in a predefined sequence; and (ii) in accordance with defined performance metrics.

One embodiment provides a system wherein the defined performance metrics include tempo of movement.

One embodiment provides a system wherein the device configuration data causes the device to provide physical output representative of the tempo of movement.

One embodiment provides a system wherein the devices execute the selected playlist asynchronously in response to an input provided by an individual local user.

One embodiment provides a system wherein the devices execute the selected playlist synchronously in response to an instruction delivered via the system.

One embodiment provides a method performed a system as described herein.

One embodiment provides a method performed by a workout machine based on data provided by a system described herein.

Reference throughout this specification to "one embodiment", "some embodiments" or "an embodiment" means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

In the claims below and the description herein, any one of the terms comprising, comprised of or which comprises is an open term that means including at least the elements/features that follow, but not excluding others. Thus, the term comprising, when used in the claims, should not be interpreted as being limitative to the means or elements or steps listed thereafter. For example, the scope of the expression a device comprising A and B should not be limited to devices consisting only of elements A and B. Any one of the terms including or which includes or that includes as used herein is also an open term that also means including at least the elements/features that follow the term, but not excluding others. Thus, including is synonymous with and means comprising.

As used herein, the term "exemplary" is used in the sense of providing examples, as opposed to indicating quality. That is, an "exemplary embodiment" is an embodiment provided as an example, as opposed to necessarily being an embodiment of exemplary quality.

### BRIEF DESCRIPTION OF THE DRAWINGS

Embodiments of the invention will now be described, by way of example only, with reference to the accompanying drawings in which:
FIG. 1A provides a top view of a device according to one embodiment.
FIG. 1B provides a top view of a device according to one embodiment.
FIG. 2A provides a side view of a device according to one embodiment.
FIG. 2B provides a side view of a device according to one embodiment.
FIG. 3 illustrates a processing unit optionally used by the device of FIG. 1A or FIG. 1B.
FIG. 4 illustrates a technology framework according to one embodiment.

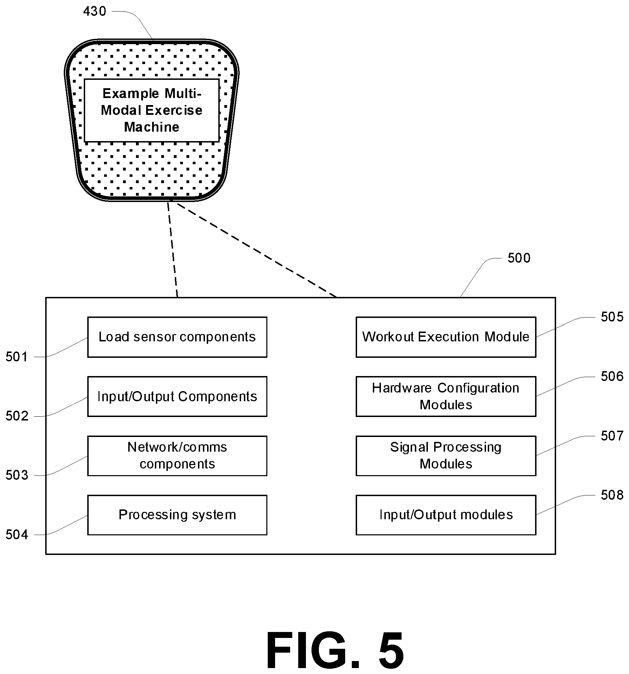

FIG. 5 illustrates an example workout device according to one embodiment.
FIG. 6A illustrates a method according to one embodiment.

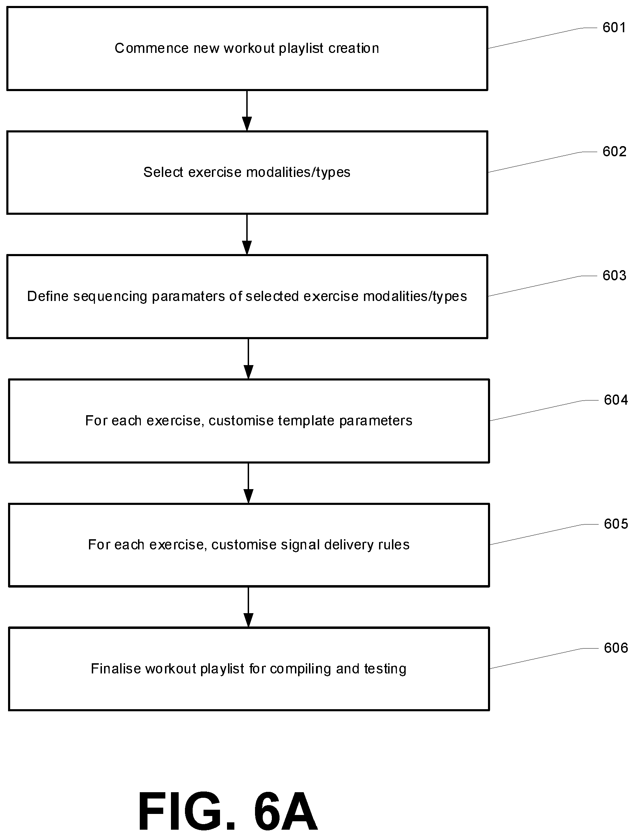

FIG. 6A illustrates a method according to one embodiment.

### DETAILED DESCRIPTION

The present disclosure relates, in various embodiments, to a sensor-enabled platform device configured to measure athletic activity. For example, some embodiments provide a platform which is configured to enable functional workouts, for example including medicine ball workouts, cardiovascular workouts, and/or resistance training, with the sensors being configured to measure participant performance in performing associated exercises. Although the present invention is described primarily in connection with such examples, it will be appreciated that further embodiments find wider application.

In overview, various embodiments include a platform device including a platform having an upper surface which is configured to enable measurement of an applied force. For example, in some embodiments, this upper surface is configured such that a user is enabled to repeatedly impact a portable object (such as a medicine ball) onto that upper surface. The surface is preferably large enough to allow for a user to stand on the surface and perform exercises such as stationary running and/or skipping. In this regard, the upper surface is preferably substantially planar, and orientated in a substantially horizontal plane. One or more sensors configured to monitor force applied to the upper surface of the platform. These are arranged such that the sensors are configured to monitor metrics associated with one or more varieties of physical exercise. For example, in some embodiments the one or more varieties of physical exercise include one or more of the following:
- The impacting of the portable object onto the surface, for example an impact force associated with the impacting of the object onto the surface. This may include a portable object such as a medicine ball.
- The impacting of body weight onto the surface, for example an impact force associated with body weight being applied during an exercise such as skipping, stationary running, jumping, burpees, and the like.
- A combination of body weight and resistance being applied through the surface. For example, this may include an implementation where a user stands on the surface, and interacts with resistance bands (i.e. a tether configured to provide resistive force) attached the platform at a location de-coupled from the surface such that, when stretched, additional resistance force associated with the stretching is applied to the surface. In some embodiments the platform is configured to measure time series data representative of resistance force, based on an excess force above body weight applied through the use of a resistance tether (the resistive strength of which may be known by the platform device software, or unknown)/
- Combinations of the above.

It will be appreciated that these are examples only, and the hardware described herein is optionally used in a variety of other ways.

The sensors are coupled to a processing unit, which is configured to process data received from the one or more sensors, thereby to derive metrics representative of athletic performance associated with impacting a portable object onto the upper surface. These metrics optionally include:
- A metric representative of force associated with a given event. The term "event" describes a set of data, for example time series force data, which includes a peak surrounded by two troughs. Such an event may be representative of an impact event (e.g. a medicine ball impact), a human movement event (for example a jump in the context of skipping, or a step in the context of stationary running), a resistance training event (for example a resistance training repetition defined by a start point, resistance movement, and return to start point).
- Metrics derived from time series force data during an event, for example first and/or second order derivative data. This may be used to assess the explosiveness of movements, control during resistance training movements, repetition rates, and the like.
- A metric representative of cumulative force during a workout period. For example, in the context of impact workouts, such as medicine ball workouts, this may measure a total amount of impact force generated. First and/or second order derivatives may also be used, for example to allow calculation of work as a function of time during a workout.
- A metric representative of average force during a workout period. For example, this average is calculated across a plurality of events.

A range of other metrics may also be calculated.

In this manner, the device is enabled to allow for measurement and monitoring of athletic performance during a range of workouts, including any one or more of: (i) object-impacting workouts, for example a medicine ball workout (also known as a "slam ball" workout), a hammer slamming workout, and the like; (ii) body weight workout such as skipping, stationary running, and/or jumping; and (iii) resistance workouts, for example resistance exercises using resistance bands.

For the sake of embodiments described below, reference is made to a particular example is an impact workout using a portable object in the form a medicine ball (with a "medicine ball" being in essence an object that is configured to be forcibly dropped with little or no rebound, for example a sand-filled bladder). This is an example only. In some embodiments the platform device is configured to provide only for impact workouts (for instance only medicine ball workouts); in other embodiments the platform device additionally/alternately provides for body weight and/or resistance workouts.

Three main categories of embodiment are considered herein.

The first category of embodiments provide a split platform, which includes: (i) a first set of one or more sensors configured to monitor force applied to a first region of the platform; and (ii) a second set of one or more sensors configured to monitor force applied to a second region of the platform. Typically, the first region of the platform represents an impact target region onto which the object is impacted during normal use, and the second region of the platform represents a standing region on which a user stands during normal use. This allows for segregation of force measurements which transfer through the user's feet, and force measurements which are specifically associated with the impact of the ball.

The second category of embodiments include a split platform, which includes one or more sensors configured to monitor force applied to a first region of the platform, represents an impact target region onto which the object is impacted during normal use, and a second region of the platform represents a standing region on which a user stands during normal use (which is not monitored by force sensors).

The third category of embodiments include a one-piece platform, which provides both an impact target region of the platform onto which the object is impacted during normal use, and a standing region of the platform on which a user stands during normal use (these may be only notionally defined, and not distinguishable by any stricture or feature, only by function; in some cases markings may be applied to the surface to identify a target region to assist with exercises). The one or more sensors are configured to monitor force applied to the upper surface of the one-piece platform, such that they are configured to measure force applied both an impact target region of the platform onto which the object is impacted during normal use, and a standing region of the platform on which a user stands during normal use. It will be appreciated that digital signal processing techniques may be applied to determine metrics of an impact, and optionally metrics associated with the weight of the user, and the weight of the object (noting that, during a medicine ball slamming movement, there is a period of time during which the mass of the medicine ball is separated from the platform). For example, the processing unit is configured to process a time domain signal thereby to determine a weight of the object. This time domain signal may also be processed to determine additional metrics associated with performance, for example including a metric representative of the "explosiveness" of ball lifting. However, it will be appreciated that this category of embodiment is best suited for a range of exercise types, including body weight exercises and resistance exercises. Specifically, digital signal processing of time series force data applied to the surface is able to be processed thereby to extract metrics relevant to a wide range of exercise types.

The metrics representative of athletic performance include metrics derived from sensing of data representative of force with which the portable object is impacted on the upper surface. In the context of a medicine ball workout, these metrics may include any one or more of the following:
- Workout average impact force.
- Workout cumulative impact force.
- Workout average repetitions-per-minute.

In some cases, one or more metrics are normalised based on the weight of a medicine ball being used, and/or the weight of the user. This is in some cases inferred from sensor data (e.g., via processing of a time-domain signal), and in other cases determined via specific input.

The upper surface may be textured or the like; the term "substantially planar" is used to indicate a generally flat region onto which an object such as a medicine ball is able to be forcibly dropped. In some embodiments the upper surface is configured substantially in a horizontal plane. However, a tilted surface may be used (for example to assist in return on the medicine ball to a user).

The one or more sensors configured to monitor force applied to the upper surface of the platform include at least one sensor configured to measure a metric associated with a force of the object impacting the upper surface. For example, these may include various forms of compression force transducers, load cell sensors, strain gauges, and the like. It will be appreciated that a selection of sensor will be dependent on specific design aspects, for example location of sensors and/or configuration of a platform support. Multiple sensors may be positioned at distinct locations.

Some embodiments make use of compression force sensor, for example sensors configured for compression force measurement with full-bridge, bonded foil strain gauge technology. These provide excellent long-term stability and ensure high performance even in applications requiring in excess of 1 million load cycles

Some embodiments make use of strain gauges, for example using MEMS sensor technology. In such technology, the pressure of media is measured by a silicon or silicon-on-insulator sensing element that has the piezoresistive strain gage bridge on it, with a transfer medium, typically silicon oil, present between the MEMS element and a stainless-steel diaphragm. The pressure exerted on the diaphragm is transferred to the MEMS element through the oil. The change in pressure results in a change in electrical output from MEMS elements piezoresistive strain gage. This electrical output gives us an indication of the pressure changes within the system.

Some embodiments make use of thin film sensors. In thin film sensor technology, a piezoresistive strain gauge bridge is deposited directly on the cell's stainless-steel diaphragm which is in direct contact with the media being measured. With this technology, there is no transfer medium in between the media and sensing bridge measuring the pressure. This eliminates the damaging expansion or shrinkage that can occur in a MEMS device.

Regardless of the sensor used, the processing unit is configured to process a sensor signal which is representative of an impact force applied to the platform, for example being an impact force created by a user forcibly dropping/throwing a medicine ball onto the platform. Preferably, the processing unit is configured to perform a normalisation process thereby to account for the weight of the medicine ball. For example, this may include subtracting ball weight from the impact force. Normalisation based on ball weight may be achieved via any of the following approaches:

In some embodiments, a sensor arrangement is configured to measure force applied to a platform which is used for both standing (or assuming other poses which place all of a portion of body weight force into the platform) and object impacting. As such, this sensor arrangement provides time domain data which varies through an athletic movement (event). For example, in the case of a medicine ball workout, this includes: (i) a period of time when the mass of the medicine ball is detectable by the sensor arrangement, for example as it rests on the platform or is held by the user; and (ii) a period of time when the medicine ball is airborne, and hence its mass is not detectable by the sensor arrangement. Digital signal processing techniques are used to determine combined user/ball weight, user-only weight, and ball-only weight. In the case of a body weight exercise, time domain data regarding an event may include data representative a period of time during which a magnitude of force detected by the sensor progresses from a minimum, to a maximum, and back to a minimum. Rates of movement, rates of acceleration of movement, and time held at peak may all be relevant metrics. In the case of a body weight exercise, the time domain data may be processed to extract force and time metrics associated with the user pushing away from the platform (e.g. initiating a jump) and landing back on the platform. This may be processed to predict jump height, explosiveness, and the like.

In some embodiments, the device includes a weight sensor, configured to measure weight of a medicine ball in isolation (that may be the same sensor used for measuring athletic performance). This may be integrated into the platform device, so that a user rests the ball on the platform initially.
- In some embodiments, a user stands on a secondary platform, which contains a separate force sensor. This allows the processing unit to determine weight of a user, weight of a user with the ball, and hence weight of the ball. It will be appreciated that the measured weight varies throughout usage as the user lifts and drops the ball.
- In some embodiments a user manually enters ball weight via a user interface.
- In some embodiments a ball includes a machine-readable token (e.g., a barcode or RFID tag) which is representative of weight,

The processing unit is preferably coupled to a display screen. This display screen may be part of the device (for example a screen provided on a vertically extending member which is mounted to a base member of the device), or a remote display screen (for example connected via a wired or wired configuration. The processing unit is also coupled to a user interface device, which may include a series of buttons, and/or a touch screen element provided in conjunction with the display screen.

The vertically extending member illustrated in the diagrams is exemplary only. Preferably, such a member is configured thereby to minimise risks of a user inadvertently slamming the medicine ball into a display screen and/or display screen support. In some embodiments a display is provided on/adjacent/below platform 102, and/or projected onto such a location.

The processing unit is configured to cause the display screen to display metrics derived from the motion sensors. This may include any one or more of the following, and/or metrics derived from any one or more of the following:
- Impact force (for impact exercises);
- Lifting force (for resistance exercises);
- Time under Force/Tension (for resistance exercises)
- Cumulative impact force;
- Effort, derived from impact force normalised for object mass;
- Repetition rate;
- Average impact force;
- Average effort;
- Object acceleration;
- Lift force metrics;
- Object acceleration;
- Total mass lifted;
- Calories burned; and
- A performance metric based on object mass and user mass.

In some embodiments, the processing unit is configured to measure and cause displaying of a metric based on a process including: (i) determining a mass of the portable object; (ii) determining an impact force at which the portable object strikes the platform; and (iii) based on (i) and (ii), deriving a metric for effort associated with striking the object on the platform.

The processing unit, in response to commands inputted via the user interface device, is configured to execute software instructions representative of one or more predefined workout programs, thereby to, via a display screen: (i) instruct a user to perform athletic activities; and (ii) display data derived from the one or more sensors thereby to provide feedback data regarding athletic performance in a current workout program.

In some embodiments the device includes a multi-user interface configured to enable display of relative operation of two or more of the platform devices having a substantially planar upper surface and a respective one or more sensors configured to monitor force applied to the upper surface of the platform. For example, multiple devices can be linked together, with a single display screen presenting metrics. This optionally display of competitive metrics for a plurality of simultaneous users.

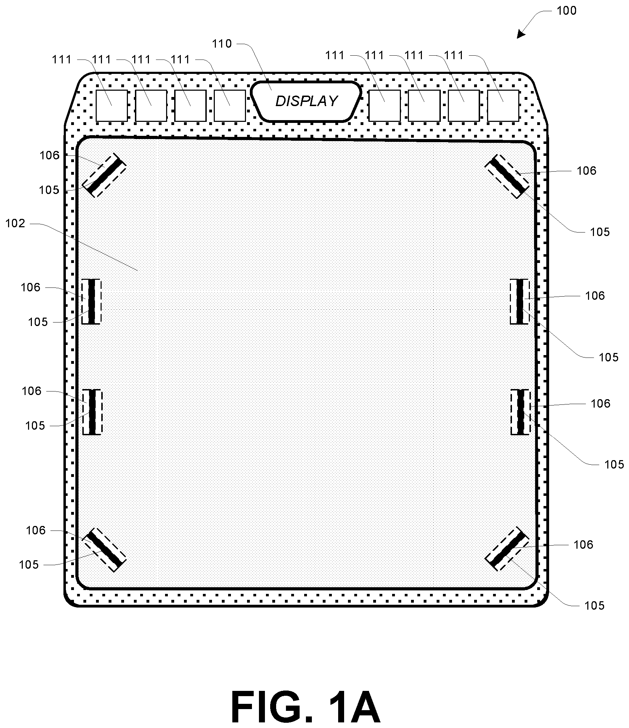

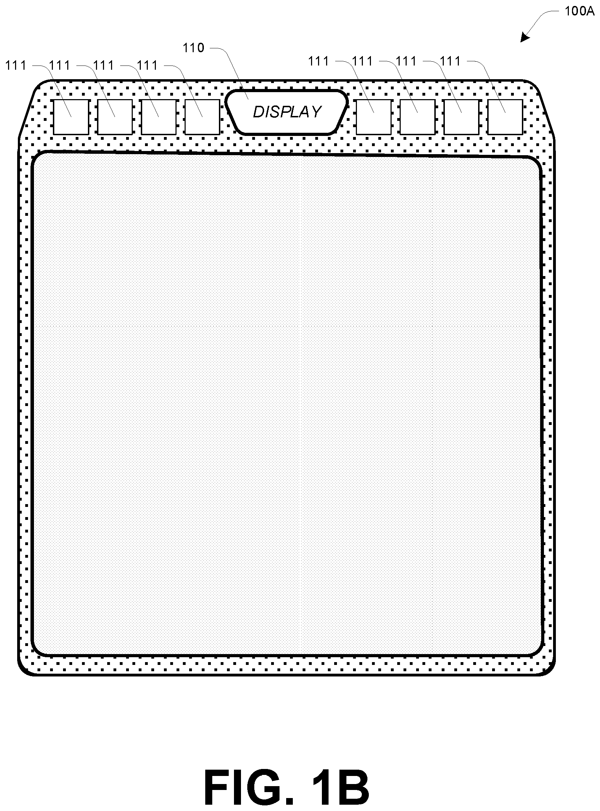

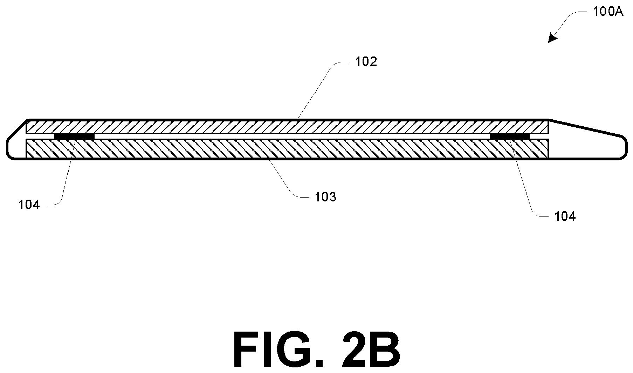

FIG. 1A, FIG. 2A, and FIG. 3 provide illustrations of an example platform device 100 according to one embodiment (a top view, side partial cutaway view, and functional diagram of processing components). FIG. 1B and 2B illustrate an alternate embodiment.

Device 100 is a standalone piece of electronic athletic training equipment, configured to enable a wide range of functional workouts. Device 100 includes a platform 102 with which a user interacts for the purpose of such workouts. Platform 102 includes an upper surface which is substantially planar, optionally including texturing via a non-slip rubber covering and/or a resilient rubber matting. The platform is preferably about 1m x 1m, allowing ample space for a user to stand and perform various forms of functional training exercises (for example squats, medicine ball workouts, resistance band movements, and the like).

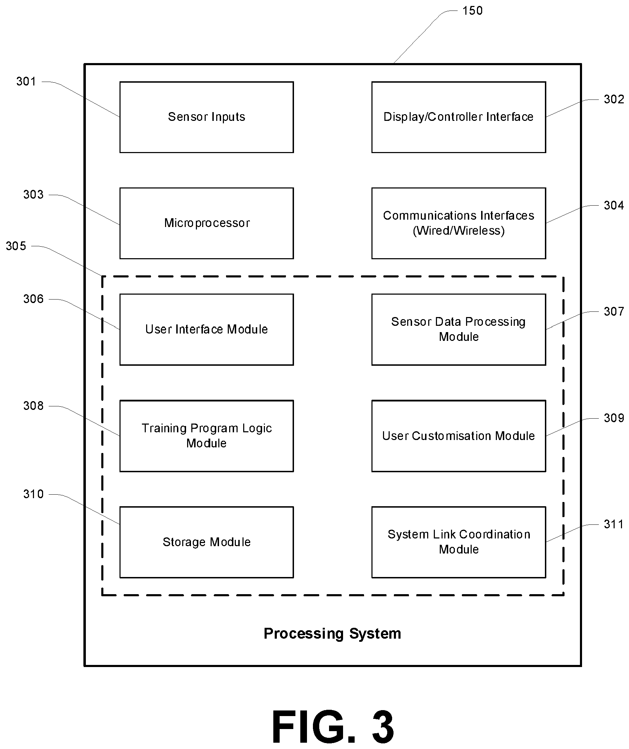

Platform 102 is mounted to a base member 103, which are in the present embodiment connected by way of a plurality of load cells 104. These load cells are configured to measure a force applied between platform 102 and base member 103. The location and number of load calls varies between embodiments. For example, in some embodiments between three and ten load cells are used, located a short distance inward of a periphery of platform 102, spaced around the periphery, and/or at more central locations. Force measurements at the various load cells are preferably combined, for example via an averaging algorithm, thereby to derive data representative of a force applied downwardly on platform 102. This is preferably recorded as time-series data (i.e. force as a function of time) in a processing system 150, for example as shown in FIG. 3. In this regard, motion (or compressive force) of platform 102 relative to base member 103 may be used to measure force exerted into platform 102. This allows for monitoring (and measurement) of athletic workouts performed on platform 102. Examples include:
- Body weight exercises, for example stationary running, squats, push ups (with only hands on platform 102), burpees, skipping, jumps, and the like. In some examples, processing system 150 is configured to identify that individual repetitions of an exercise are performed (for example an individual push up, jump or skip). In some examples processing system 150 is additionally configured to measure performance metrics for each repetition, for example based on data attributes extracted from time series force data. This allows, for instance, derivation of metrics representative of jump height and the like.
- Object impact exercise, for example medicine ball workouts. In such exercises, a user interacts with an object, and impacts that object against platform 102 (in the present examples whilst also standing on platform 102). Again, processing system 150 is configured to identify and count repetitions, and preferably performance metrics (for example impact force).

The load cells may optionally be a GML654 Galoce Robotic Tactile Sensing Load Cell, or similar device, for example with a 20 KN rating. Those skilled in the art will recognise how appropriate load cells are selected based on attributes such as resolution, dynamic range, and sampling rate. Multiple different load cell types bay be used, with one type selected for optimal sensitivity (to assist in identifying specific motions), and another for optimal sample rate (which may be relevant in accurately identifying peak impacts). In further embodiments, means other than load cells are used to measure force applied to platform 102. This may include technologies which measure strain in the platform device.

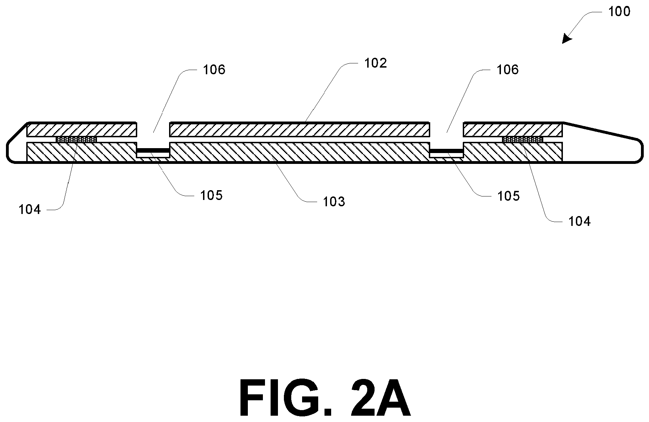

In the example of FIG. 1A and FIG. 2A, base member includes a plurality of connector members 105, which are located beneath apertures 106 in platform 102. Each connector member is configured to enable connection of a resistance band, for example via a carabiner or the like. For example, in some embodiments each connector member include a loop, latch, cylindrical bar, or the like. The number and location of connector members 105 and apertures 106 varies between embodiments, with the positioning shown in FIG. 1A being an example only. Preferably at least two connector members 105 are provided. These connector members 105 may be provided on base member 103 peripherally outward of platform 102, negating the need for apertures 106. In any event, connector members 105 allows the user to perform resistance exercises via device 100. Tension between the user and one or more resistance bands increases force between platform 102 and base 103, which is observed in load cells 104. This allows for processing unit 105 to identify exercise repetitions, and determine performance metrics of those repetitions (for example amount of work being performed, time under tension, rate of movement, and so on).

Connector members 105 are preferably provided as opposed pairs, thereby to allows a resistance band for each side of the body. Providing multiple pairs as shown in FIG. 1A allows for a greater range of exercise options, including use of multiple connection points for each resistance band (e.g. to cross over a user's back during resistance push-ups). Furthermore, using multiple connection points on a single resistance band allows for resistance characteristics of a that band to be adjusted, rather then needing to switch between bands or different ratings.

Device 100, as illustrated, also includes a display 110 (for example an LED display). Display 110 is coupled to processing system 150, and enables for the display of a range of information. This may include, for example:
- Workout instructions. For example, a user is provided with instructions on an exercise that is to be performed, and/or special steps to be undertaken. This is particularly relevant for workout playlists as discussed further below,. whereby a user undertakes a workout involving multiple different exercise types.
- A user interface with selection means. In some embodiments device 100 includes input means, for example buttons or the like, thereby to facilitate interaction with controls and display 110. In one embodiment, platform 102 serves as an input, with different locations (e.g. corners) operating as quasi-buttons, with processing unit 150 determining input locations via comparative load cell data values.
- Live workout metrics. For example, this may include repetition count, repetition rates, calories burned, calorie burn rate, repetition impact force, average impact force, cumulative impact force, resistance metrics, and so on. In some cases a user can switch between different metric display options, as is common in known workout equipment.

In some embodiments, display 110 is a secondary means for input and control of device 100, with a user instead interacting via a smartphone app executing on a mobile device coupled to device 100 via a network connection, as discussed further below.

In the illustrated embodiment, device 100 additionally includes a set of indicator lights 111. These may be substituted for a range of other output devices. These are preferably used to provide any one or more of the following outputs:
- A timer, for example showing a user their temporal progression through a workout.
- A force indicator, showing a user the amount of force they are putting into platform 102. This is preferably normalised based on user weight, so that both large and small users are able to experience a dynamic range of output values.
- A repetition counter (for example counting down a number of repetitions for a defined workout).
- A metronome for example providing a visual indicator for timing of exercises and/or exercise components. This may include, for a resistance exercise, a metronome showing a lift portion, hold portion, release portion, and rest portion.

FIG. 3 illustrates functional components of processing system 150. Processing system 150 is preferably embedded inside base 103, and includes a microprocessor, which is configured to execute software instructions thereby to perform various key functionalities of device 100. These include:
- Processing data received from load cells 104, thereby to determine data values. These may include data values representative of athletic performance.
- Delivering a user interface, via an output device. In the illustrated example, a display screen 110 is provided.
- Receiving and processing user interface inputs, for example via buttons, connected devices, through platform 102, and the like.
- Executing software instructions representative of one or more predefined workout programs, thereby to, via a display screen: (i) instruct a user to perform athletic activities; and (ii) display data derived from the one or more sensors thereby to provide feedback data regarding athletic performance in an executing one or the one or more predefined workout programs.

In some embodiments processing system 150 processes a time domain signal, and from that processing extracts data artefacts which are associated with particular signal attributes. For example, in a single platform arrangement, a time domain signal is representative of a cycle of event repetitions (e.g. impact repetitions, body movement repetitions, and/or resistance exercise repetitions). For example:
- In the case of a medicine ball workout, each repetition includes: (i) a start component, where a user stands on the platform and the ball rests on the platform; (ii) a lift component, where the user accelerates the ball upwards; (iii) a slam component, where the user accelerates the ball downwards; (iv) a transit component, where the ball is airborne; and (v) an impact component whereby the ball impacts the platform and decelerates to zero velocity.
- In the case of a body weight exercise, each repetition includes: (i) a start component where there is a nominal body weight force applied through the platform, (ii) a positive motion component where the force increases and peaks (e.g. during a jump motion); (iii) optionally a zero force component where there is no force applied to the platform (e.g. whilst the user is airborne); and (iii) a negative motion component (e.g. where the user lands from a jump).
- In the case of a resistance weight exercise, each repetition includes: (i) a start component where the force through the platform is a local minima, being a force associated with body weight and a minimum level of stretch in resistance bands; (ii) a positive motion component whereby the force progressively increases as tension in the resistance band is increased through an exercise motion; (iii) optionally a peak component, whereby the force stabilises at an approximate maximum for a period of time (e.g. a user holds in tension at the maximum position of a motion for a prolonged period); and (iv) a negative motion component whereby force progressively decreases as tension in the resistance band lessens, during a motion of return to the start position. It will be appreciated that the timing of each component of the exercise may be relevant for the purposes of instruction and/or assessing performance. In some embodiments a timer/metronome may be provided to guide movement rate.

In use, a user interacts with the user interface device thereby to activate device 100 (for example bringing device 100 out of a sleep/dormant mode). The user then selects one of the predefined workout programs, and in some cases is prompted to enter one or more data attributes (for example age). The workout program then commences, and the user participates in the workout as directed (for example by slamming the medicine ball repeatedly onto platform 102). Physical values are measured by load cells 104, and processed by processing system 150, which in response causes output via display screen 110 of workout metrics (for example repetition rate, effort metrics, and so on).

Example workout programs include:
- Medicine ball - Cumulative impact force target. A cumulative force target value is defined, and a user seeks to hit that target in the shortest time possible.
- Medicine ball - Average impact force target. An average force target value is defined, and a user seeks to maintain an average impact force above that target for the duration of a workout.
- Medicine ball - Interval training, whereby a user is instructed in respect of both a "work" period, where there is a focus on maximising impact force, and a "rest" period, where there is a focus on maintaining a threshold rate of repetitions.
- Body weight - Skipping, whereby a user is instructed to perform a skipping workout whilst standing on the platform.
- Body weight - Running, whereby a user is instructed to perform a stationary running workout whilst standing on the platform.
- Body weight - Jumps, whereby a user is instructed to perform a jumping workout whilst standing on the platform.
- Resistance - Bent Over Row, whereby a user stands on the platform, grasps two resistance bands attached to the base (or grasps a bar which connects the resistance bands at their respective proximal ends), and performs a bent over row exercise.
- Resistance - Squat, whereby a user stands on the platform, grasps two resistance bands attached to the base (or grasps a bar which connects the resistance bands at their respective proximal ends), and performs a squat exercise.
- Resistance - Bench Press, whereby a user lies with their upper back on the platform, grasps two resistance bands attached to the base (or grasps a bar which connects the resistance bands at their respective proximal ends), and performs a bench press style exercise.

It will be appreciated that these are examples only, and that a range of other workout types may be configured. This is particularly the case for resistance exercises, where different length of resistance bands and attachments may be configured to allow a wire range of workouts.

FIG. 3 illustrates functional components of an example processing system 150. Software executed by system 150 is described by reference to a plurality or "modules". The term "module" refers to a software component that is logically separable (a computer program), or a hardware component. The module of the embodiment refers to not only a module in the computer program but also a module in a hardware configuration. The discussion of the embodiment also serves as the discussion of computer programs for causing the modules to function (including a program that causes a computer to execute each step, a program that causes the computer to function as means, and a program that causes the computer to implement each function), and as the discussion of a system and a method. For convenience of explanation, the phrases "stores information," "causes information to be stored," and other phrases equivalent thereto are used. If the embodiment is a computer program, these phrases are intended to express "causes a memory device to store information" or "controls a memory device to cause the memory device to store information." The modules may correspond to the functions in a one-to-one correspondence. In a software implementation, one module may form one program or multiple modules may form one program. One module may form multiple programs. Multiple modules may be executed by a single computer. A single module may be executed by multiple computers in a distributed environment or a parallel environment. One module may include another module. In the discussion that follows, the term "connection" refers to not only a physical connection but also a logical connection (such as an exchange of data, instructions, and data reference relationship). The term "predetermined" means that something is decided in advance of a process of interest. The term "predetermined" is thus intended to refer to something that is decided in advance of a process of interest in the embodiment. Even after a process in the embodiment has started, the term "predetermined" refers to something that is decided in advance of a process of interest depending on a condition or a status of the embodiment at the present point of time or depending on a condition or status heretofore continuing down to the present point of time. If "predetermined values" are plural, the predetermined values may be different from each other, or two or more of the predetermined values (including all the values) may be equal to each other. A statement that "if A, B is to be performed" is intended to mean "that it is determined whether something is A, and that if something is determined as A, an action B is to be carried out". The statement becomes meaningless if the determination as to whether something is A is not performed.

At each process performed by a module, or at one of the processes performed by a module, information as a process target is read from a memory device, the information is then processed, and the process results are written onto the memory device. A description related to the reading of the information from the memory device prior to the process and the writing of the processed information onto the memory device subsequent to the process may be omitted as appropriate. The memory devices may include a hard disk, a random-access memory (RAM), an external storage medium, a memory device connected via a communication network, and a ledger within a CPU (Central Processing Unit).

Example processing system 150 includes a microprocessor 303, which is configured to execute software instructions 305, which are stored on a memory device. For example, this may include software which operates on an Android operating system.

A user interface module 306 is configured to cause rendering of a graphical user interface via a display screen which is coupled to processing system 150 via a display/controller interface 302. User interface module controls display of rendered content in response to programming logic and plurality of inputs, including:
- Inputs received from a user via interface 302. For example, these may include inputs provided via a touch screen, and/or one or more buttons (and/or, in some embodiments, input delivered by a user's foot input to specific locations on platform 102). Inputs may include, for example: (i) an input which triggers recovery from a "sleep" mode; (ii) an input which selects a workout program from a plurality of displayed workout programs; (iii) inputs which provide parameters relevant to customisation of the selected workout program (for example weight, time, intensity, and the like); (iv) parameter display settings; and (v) workout program controls (for example pause, stop, restart, and the like).
- Input from training program logic module 308, which maintains execution instructions for a plurality of selectable workout programs. For example, upon selection of a workout program by a user, module 308 provides instructions to the user interface module to configure display of data fields, including fields configured for pre-workout data collection prompts, fields configured to display intra-workout metrics, and fields configured to display post-workout results.
- Inputs derived from processing of sensor data received via sensor inputs 301, via a sensor data processing module 307. Module 307 is configured to receive raw inputs from connected sensors, and process those thereby to provide displayable metrics. This may include operations including rounding, averaging, normalising, combining, and the like. In the present embodiment, module 310 stores calculated values in data tables in a storage module 310. These values are used for either or both of: (i) subsequent calculations (for example where averages are calculated); and (ii) pulling of data for display in display fields rendered on the display screen under control of module 308.

In some embodiments, a user customisation module 309 allows a user to customise various functionalities of processing system 150, for example by accessing a predefined user account with historical data stored in storage module 310.

In some embodiments a system link communication module 311 is configured to provide system link functionalities, thereby to enable display of competitive metrics from a plurality of two or more devices. Module 311 is optionally configured to perform either or both of the following:
- Transmitting current workout metrics defined by module 307 and/or stored in module 400 to a remote device, which may be a shared display screen or a second similar athletic training device having similar hardware (e.g. a similar processing system 150).
- Receiving current workout metrics from an external device (for example a second similar athletic training device having similar hardware), and providing those metrics for display via user interface module 306 on the display screen.

In relation to the latter, in some embodiments module 306 and 311 interact thereby to provide user interface options to select a multi-participant workout, in which case module 306 configured the user interface to provide data display fields configured to present metrics from one or more other devices, thereby to provide comparison metrics (allowing a user of visually monitor their performance against others).

System link module 311 is coupled to one or more other devices via communications interfaces 304, which may include wired connections and/or wireless connections (e.g., Wi-Fi or Bluetooth).

In further embodiments, the platform device is configured to interact with a range of peripheral devices, including (but not limited to): biometric sensors; smart watches; smartphones; smart mirrors (for example the "MIRROR" home gym), and the like. It will be appreciated that such devices may be configured to interact with the platform device thereby to provide instruction, display performance results, and/or gather additional metrics.

In some embodiments, communications interfaces 304 are configured to enable communication with remote computing devices. For example, this may include: computing systems, thereby to enable functions such as configuration and software updates; user mobile devices, thereby to provide additional functionalities based on mobile device app integration; and wearable sensors (for example via Ant+ standard) thereby to enable heartrate and/or other metrics to be communicated to user interface module 306.

In some embodiments, where interfaces 304 are configured to enable communication with remote computing devices, device 100 is configured to be operate in conjunction with a smartphone app thereby to facilitate improved access for control and/or configuration. For example, the smartphone is used as a primary user input device for device 100.

In some embodiments either via a smartphone or directly, device 100 provides output to a Bluetooth speaker or headset, for example thereby to deliver instructions and/or provide workout feedback.

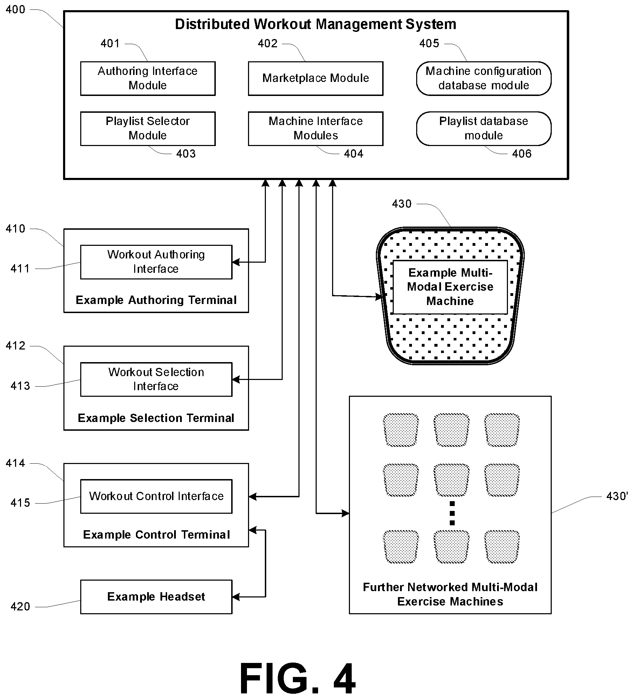

Some embodiments include a system configured to enable multiple-activity functional workouts via distributed exercise machines. An example is described below by reference to FIG. 4, which illustrates a distributed workout management system 400. System 100 may be defined by one or a plurality of server components (which may be distributed across a plurality of computing systems). For the present purposes, system 100 is illustrated and described by reference to functional components, including "modules".

The term "multi-modal workout", as described herein, refers to a workout which includes multiple different forms of human body movement, which are measured (including in terms of workout intensity/effort) via sensor components. Examples include: (i) body weight exercises, such as push ups; (ii) footwork exercises such as skipping and jumping; (iii) resistance exercises utilising resistance bands; (iv) free weight exercise, for example various kettle bell motions; and (v) impact exercise such as medicine ball. It will be appreciated that, depending on the nature of exercise machine used for the purposes of a given embodiment, other forms of human body movement may be supported.

Embodiments are described by reference to a "workout platform machine" or "platform device" as an exemplary piece of multi-modal workout hardware. It should be appreciated that this is an example only, and that the technology herein may be applied across a wide range of exercise machines which are able to be controlled thereby to deliver multi-modal workouts. An example of a "workout platform machine" is the device described above in relation to FIG. 1A to FIG. 3. In brief, "workout platform machine" is a piece of equipment which includes sensors configured to measure force applied to a platform, for example as a time series data. This data is then processed by various means, for example using digital signal processing techniques, thereby to quantify exercise metrics. For example, exercise metrics may include the likes of: (i) whether a particular exercise has been performed; (ii) a metric representative of effort for that exercise; (ii) a metric representative of cumulative effort for multiple exercise repetitions; (iii) metrics representative of tempo for an exercise repetition, or component of an exercise repetition; (iv) metrics representative of a rate at which repetitions are performed; and (v) time under tension measurement. Other metrics may be used.

Some embodiments include a system configured to enable multiple-activity functional workouts via distributed exercise machines. An example is described below by reference to FIG. 4, which illustrates a distributed workout management system 400. System 400 may be defined by one or a plurality of server components (which may be distributed across a plurality of computing systems). For the present purposes, system 400 is illustrated and described by reference to functional components, including "modules".

System 400 is configured to communicate with a plurality of remote terminals, which may include, for example, the likes of smartphones, touch screen TV's, tablets, PCs, laptops, gaming consoles and the like. Each of these terminals is configured to render a user interface which allows a user to interact with functionalities made available via system 400. For example, the rendered user interface may be provided via a locally executing software application, via web code executed within a local web browser application, or via other means. Three example categories of terminal are illustrated in FIG. 4. These are distinguished by function for the purposes of illustration. In some embodiments a common terminal (such as a smartphone) performs the role of two or more of these terminals (optionally via a single software application). The illustrated example terminals described below, with reference to their interaction with system 400:

An example authoring terminal 410, which renders a workout authoring interface. This workout authoring interface enables a user to define a "playlist" which includes a plurality of exercises of different modalities. The authoring interface preferably allows a user (for example a personal trainer or the like, or with input from a personal trainer or the like) to set parameters for each of the exercises, and/or a manner in which they are sequenced together. This is described in more detail further below. In overview, an output of the workout authoring interface is a workout playlist, which is able to be executed by a given one or more of machines 430/430'. Workout playlist data is stored by system 400 via a playlist database module 406.

System 400 provides an authoring interface module, which provides back-end functionality to authoring interface 411. This includes:
- Maintaining data representative of customisable templates for a plurality of exercise modalities. This allows a user to select an exercise modality (for example "bent over row") and customise a template by inputting values for available variables. Available variables may differ between modalities. Generic examples include number of repetitions; target work rate for each repetition; intra-repetition tempo (optionally including positive motion, negative motion, and holds); inter-repetition tempo; repetition number; exercise duration; and the like. In some cases more complex variables are set, for example variables relating to form (e.g. explosive power vs movement control).
- Enabling a user to define a sequence for customised templates. This may include ordering/numbers of sets, and optionally standard workout type characteristics (e.g. ladder progressions, Tabata style training, AMRAP sets, and the like.
- Enabling a user to define/customise rules for the delivery of audible/visual signals to a user (for example thereby to provide instructions and/or feedback). This may include a rules-based approach whereby a user selects a condition (e.g. "before commencement of exercise", "upon detection of successful repetition", "if repetition rate falls below X RPM", "when approximately 30 seconds before next modality") and a signal (for example an instruction signal with audible verbal explanation of how to perform that modality or the next modality, feedback on performance, and/or a series of lights progressing sequentially in time to a second count, beat or amount of force applied, and so on).
- Processing data generated via the authoring of customised templates, sequencing, and signal delivery rules thereby to define executable data for a workout playlist which can be delivered to one or more of machines 430/430' and executed via that/those machines.

In some embodiments the authoring interface is supplemented by or replaced by a more complex coding/development interface, which provides additional flexibility over workout authoring, feedback, and the like. For example, this may be used for development of premium content which is sold on a marketplace (discussed further below).

An example selection terminal 412, which renders a workout selection interface 412. Workout selection interface 412 enables a user to select a workout playlist from a selection of predefined workout playlists made available via a playlist database module 406. The nature of the selection interface varies between embodiments. Optional functionalities include: (i) user-based filtering, whereby a user is able to view workout playlists created by specified users; (ii) workout type filtering, whereby a user is able to search/browse workout paylists based on attributes; (iii) subscriptions, whereby a user is presented with workouts based on a subscription, for example allowing for display of "workout of the day"; and (iv) a marketplace whereby a user is enabled to browse and purchase workout playlist content, including individual playlists and/or subscriptions to playlists from a given creator. The workout selection interface is configured to enable selection of a workout, and the delivery of that workout for execution at a specified one or more of machines 430/430'. For example, this may include either or both of: (i) a user selecting a workout playlist for delivery to a specified machine, so that they can use that machine; and (ii) a user selecting a workout playlist for deliver to a plurality of specified machines, so that a plurality of users can use that workout playlist. The latter may be synchronous (e.g. a trainer configures multiple machines in a facility such that users can participate in a common workout playlist as a group) or asynchronous (e.g. a trainer sends a daily playlist to a plurality of machines associated with his/her clients).

System 400 provides a playlist selector module which facilitates searching/browsing of available workout playlists by reference to a playlist database module 406. Optionally, a marketplace module 402 allows a user to purchase premium content for a price (optionally including individual workout playlists, collections of workout playlists, and/or a subscription to workout playlists from a specific source, for example a particular personal; trainer or the like). The playlist selector module is configured to enable selection of a defined playlist. Upon selection, a machine interaction module 404 provides a playlist delivery module which is responsive to a selection via the playlist selector module thereby to cause delivery of data via a network to a defined one or more of the distributed exercises devices, thereby to configure those devices to enable execution of the selected playlist.

In terms of creating a nexus between a given workout selection interface and one or more specific workout machines 430/430', various approaches may be used. For example, each machine may have a unique code (which may for example be read optically, via NFC/BLE, via alphanumeric input, and the like), thereby facilitating identification of a machine via a selection terminal, and addressing of data to that machine via system 400. In some embodiments selection interface 413 allows a user to create a set of "favourite" machines (which are optionally able to be formed into groups), thereby to streamline a process by which a desired one or more machines are selected for the purposes of machine interaction module 404.

An example control terminal 414, which renders a workout control interface 415. This facilitates the engagement between a user and a platform machine during the execution of a workout playlist. For example, in some embodiments interface 415 provides instructions, for example audible instructions via a headset 420, to a user of a machine thereby to guide the user through physical activities associated with an executing workout playlist. Preferably interface 415 additionally communicates metrics representative of a user's performance in undertaking a workout. This may be intra-workout (for example a rules engine associates observed metrics with audible output messages, such that a user receives real-time personal trainer style input on their performance) and/or retrospective (for example a user receives input as to their performance following completion of an exercise/set/workout). In alternate embodiments, terminal 415 and/or headset 430 communicates directly with machine 430 (e.g. via Bluetooth), rather than communication routed via machine interaction modules 404 of system 400.

In combination, interfaces 411, 413 and 415 enable a process of workout playlist authoring, workout playlist selection, and workout playlist execution (including delivery of performance and group or global ranking metrics).

A key function of system 400 is the configuration of each of machines 430/430' to execute selected workout playlists. Unlike conventional media distribution arrangements (e.g. streaming of audio/video and/or distribution of media files), system 400 requires functionality to provide instructions to the individual machines such that they are configured to:
(i) Configure internal hardware thereby to collect sensor data relevant to a specific exercise modality. For example, this may include setting sensitivity thresholds for sensor components, sampling rates, hardware component activation/deactivation, and the like.
(ii) Configure processing components to process sensor data for each specific exercise modality. For example, this includes executable instructions by which an input signal from a sensor (for example a time-series signal representative of force) is processed thereby to identify data artefacts specific to a given exercise. In practical terms, this may include differentiating a push-up motion from a resistance band rowing motion, identifying components of a medicine ball lift/hold/slam motion, and so on. This is preferably used both for the purposes of identifying that an exercise has been performed (e.g. does the time-series data match a predefined exercise "fingerprint", based on DSP method and/or operation of an Al classifier) and determining exercise metrics (which will be different for each exercise/modality, and optionally include quantification of components of the time-series data based on varying processing algorithms).

In this regard, system 400 includes a machine configuration database module 405. This provides access to a database which contains a plurality of modality data sets. Each modality data set includes, for a specific exercise modality, data representative of:
(i) Device configuration data executable via a given one of the exercise machines thereby to configure that device to receive physical input representative of the specific exercise modality. For example, by referencing a particular modality set, system 400 is able to instruct a given machine with respect to how it should configured its hardware thereby to monitor a particular modality within a given workout playlist. The device configuration data is preferably stored at a cloud location, and downloaded to individual machines as needed.
(ii) Processing configuration data executable via a that one of the exercise machines thereby to configure the device to process physical input representative of the specific exercise modality when operated in accordance with the device configuration data. This allows for the machine to determine whether a given exercise repetition has been physically performed (which may include "not performed" or "incorrectly performed"), and determine metrics associated with performance (which may include non-performance and/or incorrect performance).

The processing configuration data may additionally include (or be associated with) a machine output protocol. A "machine output protocol" is a set of logic/rules which governs the manner in which output is delivered to a user during the performance of a workout playlist. For example, rules may be defined during authoring including the likes of:
- A rule to provide a signal (visual and/or audible) when a repetition is correctly performed. For example, this can be used as a counter.
- A rule to provide a signal (visual and/or audible) when a repetition is incorrectly performed. For example, this can be used to inform a user that they are performing an exercise incorrectly.

- A rule to provide a signal (visual and/or audible) when a repetition is correctly performed with metrics within a predefined range. For example, this may be used to inform a user if they are performing an exercise at a prescribed intra-repetition prescribed tempo, and/or inform a user in relation to the power/effort being exerted during a repetition.
- A rule to provide a signal (visual and/or audible) when cumulative metrics move into (or out of) a predefined range. For example, this may be used to provide feedback regarding repetition rate, inter-repetition tempo, power/effort decrease over time, and the like.
- A rule to provide a signal where metrics for a given exercise includes defined data attributes. For example, this may be used to identify incorrect form during a free-weight motion (e.g. jerky motions).
- A rule to provide a signal where a certain proportion of repetitions (or other completion metric) is achieved, thereby to trigger delivery of information/instructions for a next exercise modality in an executing workout playlist.

Via such an approach, a workout playlist is able to be authored and executed such that visual and/or audible signals are provided to a user based on monitoring of data collected via machine sensors, enabling automated instructions, adaptive feedback, and the like.

FIG. 6A illustrates an example workout playlist authoring method, for example performed based on interaction between interface 411 and module 401. It will be appreciated that this is an example only, and provided for the sake of context. Various modifications to this method are made for the purposes of further embodiments.

Block 601 represents a process including commencing generation of a new workout playlist. This may include defining a workout name/ID via the authoring interface.

Block 602 represents a process whereby the authoring interface displays available exercise modalities/types, which are preferably navigable via a search/browse/filter arrangement. A user selects desired modalities/types, and orders them into a sequence (for example by graphical manipulation of icons), as represented by block 603. The same modality/type may be selected on multiple occasions, such that it can be customised with different metrics (e.g. repetition numbers, set time, etc).

The level of granularity by which modalities/types are defined varies between embodiments, and hierarchical structures may be used. In that regard, each exercise modality/type is associated with a data set in the machine configuration database, which provides a customisable template by which that modality/type is customised by setting of parameters. So, for instance, there may be a modality type of "resistance band" with first level subtype of "bent over row" and second level sub types of "strength", "power", "cardio" and "control". Each of those is associated with a template having customisable variables. For example, "strength" may have key variables associated with requirements for objective peak load exerted via an exercise, "power" may have key variables associated with peak load and tempo, "cardio" may have key variables associated with repetition rate, and "control" may have key variables associated with intra-exercise tempo and motion smoothness.

Block 604 represents a process by which template parameters are customised for each modality/type in the defined sequence. This may include accessing default parameters. Otherwise, a user is presented with each of the available customisable parameters, and sets a variable value for each.

Block 605 represents a process including customisation of signal delivery rules. Preferably, each template has default rules, and a user modifies those (for example with custom audio recordings) and/or adds additional rules (for example custom audio when a particular data artefact is observed, for example RPM dropping below a defined threshold). In some embodiments a user is able to search for non-standard metrics which can be measured during the exercise modality/type, and define signals based on those. Signals may be both real-time instructive, and/or representative of retrospective performance (for example to allow a user to assess their performance, optionally relative to other persons who have completed the same workout and gain ranking within a group or globally).

Block 606 represents a process whereby the paylist is compiled, which includes system 400 defining device configuration data, processing configuration data, and other executable instructions which are provided to a given one or more of machines 430/430' thereby to enable those machines to deliver the workout playlist. In some embodiments this includes a function to allow for a virtual simulation of the playlist for the purposes of testing.

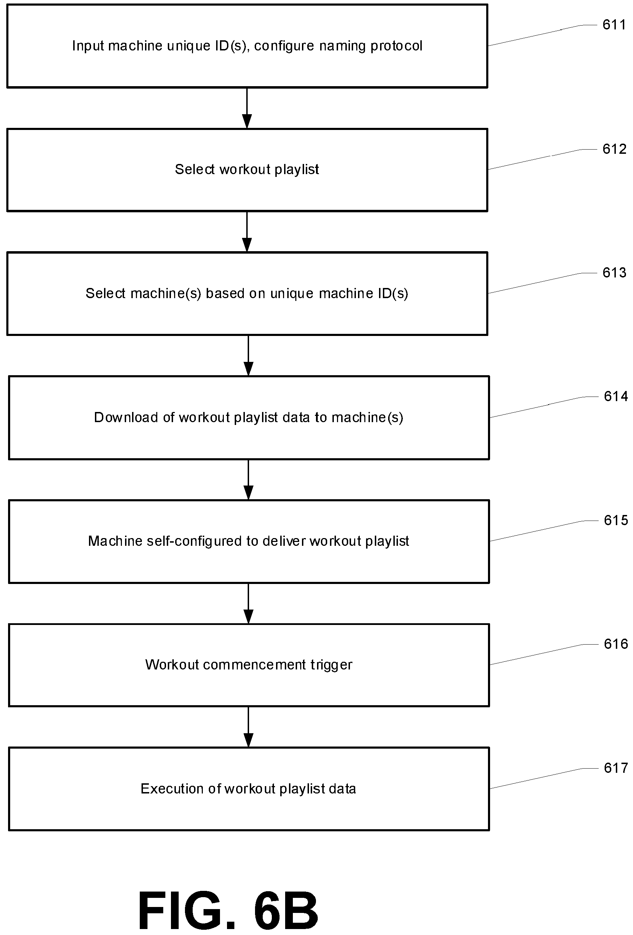

FIG. 6B sows an example method for delivering a workout playlist via one or more machines. Block 611 represents a process inputting of unique machine IDs. This may include any one or more of: manual input of an alphanumeric code; scanning of an optically readable code (such as a QR code); scanning a radio-frequency code (for example RFID or NFC); discovery via Bluetooth/BLE technologies; and other such approaches. Optionally a machine name is displayed/defined (for example based on the location of the machine, such as "GYM FLOOR #3", "GENERIC HOTEL SYDNEY, ROOM 301", or based on an owner's name). Optionally a user is able to create machine groups, such as "ALL GYM FLOOR MACHINES", or "MY REMOTE PERSONAL TRAINING CLIENTS". Block 612 represents a process of selecting a playlist, for example based on a search/browse functionality. Block 613 represents selection of a machine/machines. For example:
- In one example a user scans a machine prior to selection, and that machine is selected by default.
- In another example a user is prompted to scan a machine after selection, and that machine is selected.
- In another example a trainer user selects a group of machines, an sends the selected workout playlist to all of those machines (for example to enable communal synchronous workout in a common exercise space, or to configure multiple remote machines with a "workout of the day").

Block 614 represents downloading of data representative of the selected workout playlist to the selected machine/machines. In some cases a machine is able to locally store multiple playlists, and a user is able to select between those (or, in some embodiments, manually create their own). As represented by block 615, a machine self-configures to deliver a playlist (e.g. by configuring sensor hardware for a first exercise, and queuing initial workout instruction signals). Block 616 represents a commencement trigger, which may be an individual machine trigger (for example pressing a "start workout" button), or a remote trigger (thereby to trigger synchronous workouts across multiple machines, optionally with competitive metrics being displayed via a common screen). This commences playlist execution (block 617), including configuration/reconfiguration of hardware to monitor the various exercise modalities/types, and signal processing thereby to define workout metrics and deliver feedback/instructions.

It should be appreciated that the above disclosure provides advances in the field of workout equipment. For example, it allows medicine ball workouts to me measured and quantified (and optionally also other forms of workouts that are able to be quantified via sensors provided on a device as disclosed herein).

It should be appreciated that in the above description of exemplary embodiments of the disclosure, various features of the disclosure are sometimes grouped together in a single embodiment, FIG., or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description are hereby expressly incorporated into this Detailed Description, with each claim standing on its own as a separate embodiment of this invention.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

Similarly, it is to be noticed that the term coupled, when used in the claims, should not be interpreted as being limited to direct connections only. The terms "coupled" and "connected," along with their derivatives, may be used. It should be understood that these terms are not intended as synonyms for each other. Thus, the scope of the expression a device A coupled to a device B should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

## Claims

1. A device (100) configured to measure athletic activity, the device including:
a platform (102) having an upper surface configured to receive an applied force;
a base member (103) coupled to the platform;
a plurality of load cells (104) configured to monitor the force applied to the upper surface of the platform based on compressive force applied between the platform and the base member;a processing unit (150) configured to process data received from the plurality of load cells, thereby to derive metrics representative of athletic performance associated with a predefined category of exercise which applies the force to the upper surface, wherein the processing unit is configured to derive metrics representative of athletic performance for a plurality of distinct exercise types; a plurality of apertures (106) formed in the platform through the upper surface;
**characterised in that** the platform (102) is mounted to the base member (103) by way of the plurality of load cells, wherein:
(i) the base member (103) includes a plurality of connector members (105);
(ii) the connector members are located beneath apertures (106) in the platform (102);
(iii) each connector member is configured to enable connection of a resistance band; such that, when a user standing on the platform applies tension through a resistance band coupled to one or more of the connector members, tension between the user and one or more of the resistance bands increases force between the platform (102) and the base member (103), which is observed in the load cells (104), thereby allowing the processing unit to identify exercise repetitions and determine performance metrics of those repetitions.

2. A device according to claim 1 wherein the plurality of distinct exercise types include two or more of: (i) body weight exercised; (ii) resistance band exercises; (iii) object impacting exercises, being object impacting exercises wherein the user throws an object against the upper surface of the platform whilst the user is standing on the platform.

3. A device according to any preceding claim wherein the processing unit is configured to determine, based on data from the plurality of load cells, a metric associated with a force of an object impacting the upper surface.

4. A device according to any preceding claim wherein the processing unit is configured to determine, based on data from the plurality of load cells, a metric associated with a force of a body weight exercise being performed by a user standing or lying on the upper surface.

5. A device according to any preceding claim wherein the plurality of load cells comprises between three and ten load cells spaced around a periphery of the platform a short distance inward of the periphery of the platform.

6. A device according to claim 5 wherein the plurality of load cells comprises between three and ten load cells located at one or more central locations and spaced around a periphery of the platform a short distance inward of the periphery of the platform.

7. A device according to any preceding claim wherein the metrics representative of athletic performance include a measure representative of cumulative force over a specified time period.

8. A device according to any preceding claim wherein the metrics representative of athletic performance include a measure representative of average impact force over a specified time period.

9. A device according to any preceding claim data from the plurality of load cells are recorded as time domain information representative of the applied force.

10. A device according to claim 9 wherein the metrics are derived from the time domain information, and wherein the predefined category of exercise is an exercise performed repetitively as repetitions, and wherein the time domain information is processed thereby to identify and assess individual repetitions.

11. A device according to any preceding claim, wherein the connector members are located beneath apertures formed in the upper surface.

12. A device according to any preceding claim wherein each connector member includes a loop, latch, or cylindrical bar to enable connection of the resistance bands.

13. A device according to any preceding claim wherein the connector members are provided as opposed pairs, thereby to allow a resistance band for each side of the body.

14. A device according to any preceding claim wherein the connector members include multiple opposed pairs of the connector members.

15. A device according to any preceding claim including a set of indicator lights (111) which provide output in the form of force indicators for an amount of force a user applies to the platform

## Patentansprüche

1. Vorrichtung (100), die dazu ausgebildet ist, sportliche Aktivität zu messen, die Vorrichtung beinhaltend:
eine Plattform (102) mit einer Oberseite, die dazu ausgebildet ist, eine aufgebrachte Kraft aufzunehmen;
ein Basiselement (103), das mit der Plattform gekoppelt ist;
eine Mehrzahl von Kraftmessdosen (104), die dazu ausgebildet sind, die auf die Oberseite der Plattform aufgebrachte Kraft auf der Grundlage einer zwischen der Plattform und dem Basiselement aufgebrachten Druckkraft zu überwachen; eine Verarbeitungseinheit (150), die dazu ausgebildet ist, von der Mehrzahl von Kraftmessdosen empfangene Daten zu verarbeiten, um daraus Metriken abzuleiten, die für eine sportliche Leistung repräsentativ sind, die mit einer vordefinierten Übungskategorie verknüpft ist, welche die Kraft auf die Oberseite aufbringt, wobei die Verarbeitungseinheit dazu ausgebildet ist, Metriken abzuleiten, die für eine sportliche Leistung für eine Mehrzahl unterschiedlicher Übungsarten repräsentativ sind; eine Mehrzahl von Öffnungen (106), die in der Plattform durch die Oberseite hindurch ausgebildet sind;
**dadurch gekennzeichnet, dass** die Plattform (102) mittels der Mehrzahl von Kraftmessdosen an dem Basiselement (103) montiert ist, wobei:
(i) das Basiselement (103) eine Mehrzahl von Verbindungselementen (105) beinhaltet;
(ii) die Verbindungselemente unterhalb von Öffnungen (106) in der Plattform (102) angeordnet sind;
(iii) jedes Verbindungselement dazu ausgebildet ist, das Anschließen eines Widerstandsbands zu ermöglichen;
sodass, wenn ein auf der Plattform stehender Benutzer über ein mit einem oder mehreren der Verbindungselemente gekoppeltes Widerstandsband Spannung ausübt, eine Spannung zwischen dem Benutzer und einem oder mehreren der Widerstandsbänder eine Kraft zwischen der Plattform (102) und dem Basiselement (103) erhöht, die in den Kraftmessdosen (104) beobachtet wird, wodurch der Verarbeitungseinheit ermöglicht wird, Übungswiederholungen zu identifizieren und Leistungsmetriken dieser Wiederholungen zu bestimmen.

2. Vorrichtung nach Anspruch 1, wobei die Mehrzahl unterschiedlicher Übungsarten zwei oder mehr der Folgenden beinhaltet: (i) Körpergewichtsübungen; (ii) Widerstandsbandübungen; (iii) Übungen mit auftreffenden Gegenständen, wobei es sich um Übungen mit auftreffenden Gegenständen handelt, bei denen der Benutzer einen Gegenstand gegen die Oberseite der Plattform wirft, während der Benutzer auf der Plattform steht.

3. Vorrichtung nach einem vorhergehenden Anspruch, wobei die Verarbeitungseinheit dazu ausgebildet ist, auf der Grundlage von Daten von der Mehrzahl von Kraftmessdosen eine Metrik zu bestimmen, die mit einer Kraft eines auf die Oberseite auftreffenden Gegenstands verknüpft ist.

4. Vorrichtung nach einem vorhergehenden Anspruch, wobei die Verarbeitungseinheit dazu ausgebildet ist, auf der Grundlage von Daten von der Mehrzahl von Kraftmessdosen eine Metrik zu bestimmen, die mit einer Kraft einer Körpergewichtsübung verknüpft ist, die von einem auf der Oberseite stehenden oder liegenden Benutzer ausgeführt wird.

5. Vorrichtung nach einem vorhergehenden Anspruch, wobei die Mehrzahl von Kraftmessdosen zwischen drei und zehn Kraftmessdosen umfasst, die um einen Umfang der Plattform herum in einem kurzen Abstand einwärts des Umfangs der Plattform beabstandet sind.

6. Vorrichtung nach Anspruch 5, wobei die Mehrzahl von Kraftmessdosen zwischen drei und zehn Kraftmessdosen umfasst, die an einer oder mehreren zentralen Stellen angeordnet und um einen Umfang der Plattform herum in einem kurzen Abstand einwärts des Umfangs der Plattform beabstandet sind.

7. Vorrichtung nach einem vorhergehenden Anspruch, wobei die für eine sportliche Leistung repräsentativen Metriken ein Maß beinhalten, das für eine kumulative Kraft über einen bestimmten Zeitraum repräsentativ ist.

8. Vorrichtung nach einem vorhergehenden Anspruch, wobei die für eine sportliche Leistung repräsentativen Metriken ein Maß beinhalten, das für eine mittlere Aufprallkraft über einen bestimmten Zeitraum repräsentativ ist.

9. Vorrichtung nach einem vorhergehenden Anspruch Daten von der Mehrzahl von Kraftmessdosen werden als Zeitbereichsinformationen aufgezeichnet, die für die aufgebrachte Kraft repräsentativ sind.

10. Vorrichtung nach Anspruch 9, wobei die Metriken aus den Zeitbereichsinformationen abgeleitet werden und wobei die vordefinierte Übungskategorie eine Übung ist, die wiederholt als Wiederholungen ausgeführt wird, und wobei die Zeitbereichsinformationen verarbeitet werden, um dadurch einzelne Wiederholungen zu identifizieren und zu bewerten.

11. Vorrichtung nach einem vorhergehenden Anspruch, wobei die Verbindungselemente unterhalb von in der Oberseite ausgebildeten Öffnungen angeordnet sind.

12. Vorrichtung nach einem vorhergehenden Anspruch, wobei jedes Verbindungselement eine Schlaufe, einen Riegel oder einen zylindrischen Stab beinhaltet, um das Anschließen der Widerstandsbänder zu ermöglichen.

13. Vorrichtung nach einem vorhergehenden Anspruch, wobei die Verbindungselemente als gegenüberliegende Paare bereitgestellt sind, um dadurch ein Widerstandsband für jede Körperseite zu ermöglichen.

14. Vorrichtung nach einem vorhergehenden Anspruch, wobei die Verbindungselemente mehrere gegenüberliegende Paare der Verbindungselemente beinhalten.

15. Vorrichtung nach einem vorhergehenden Anspruch, beinhaltend einen Satz von Anzeigeleuchten (111), die eine Ausgabe in Form von Kraftanzeigen für einen Betrag einer Kraft bereitstellen, die ein Benutzer auf die Plattform aufbringt.

## Revendications

1. Dispositif (100) configuré pour mesurer l'activité athlétique, le dispositif comprenant :
une plateforme (102) ayant une surface supérieure configurée pour recevoir une force appliquée ;
un élément de base (103) couplé à la plateforme ;
une pluralité de cellules de charge (104) configurées pour surveiller la force appliquée à la surface supérieure de la plateforme sur la base de la force de compression exercée entre la plateforme et l'élément de base ; une unité de traitement (150) configurée pour traiter des données reçues à partir de la pluralité de cellules de charge, afin d'obtenir des mesures représentatives de la performance athlétique associées à une catégorie d'exercice prédéfinie qui applique la force à la surface supérieure, dans lequel l'unité de traitement est configurée pour obtenir des mesures représentatives de la performance athlétique pour une pluralité de types d'exercices distincts ; une pluralité d'ouvertures (106) formées dans la plateforme à travers la surface supérieure ; **caractérisé en ce que** la plateforme (102) est montée sur l'élément de base (103) au moyen de la pluralité de cellules de charge, dans lequel :
(i) l'élément de base (103) comporte une pluralité d'éléments de connexion (105) ;
(ii) les éléments de connexion sont situés sous les ouvertures (106) dans la plateforme (102) ;
(iii) chaque élément de connexion est configuré pour permettre la connexion d'une bande de résistance ;
de sorte que, lorsqu'un utilisateur se tenant debout sur la plateforme applique une tension à travers une bande de résistance couplée à un ou plusieurs des éléments de connexion, la tension entre l'utilisateur et une ou plusieurs des bandes de résistance augmente la force entre la plateforme (102) et l'élément de base (103), ce qui est observé dans les cellules de charge (104), permettant ainsi à l'unité de traitement d'identifier les répétitions d'exercice et de déterminer les mesures de performance de ces répétitions.

2. Dispositif selon la revendication 1 dans lequel la pluralité de types d'exercices distincts comporte deux ou plus des éléments suivants : (i) exercices au poids du corps ; (ii) exercices avec bande de résistance ; (iii) exercices d'impact d'objet, étant des exercices d'impact d'objet dans lequel l'utilisateur jette un objet contre la surface supérieure de la plateforme alors qu'il se tient debout sur celle-ci.

3. Dispositif selon une quelconque revendication précédente, dans lequel l'unité de traitement est configurée pour déterminer, sur la base de données provenant de la pluralité de cellules de charge, une mesure associée à une force d'un objet impactant la surface supérieure.

4. Dispositif selon une quelconque revendication précédente, dans lequel l'unité de traitement est configurée pour déterminer, sur la base de données provenant de la pluralité de cellules de charge, une mesure associée à une force d'un exercice au poids du corps étant réalisé par un utilisateur se tenant debout ou allongé sur la surface supérieure.

5. Dispositif selon une quelconque revendication précédente, dans lequel la pluralité de cellules de charge comprend entre trois et dix cellules de charge espacées autour d'une périphérie de la plateforme à une courte distance vers l'intérieur de la périphérie de la plateforme.

6. Dispositif selon la revendication 5, dans lequel la pluralité de cellules de charge comprend entre trois et dix cellules de charge situées au niveau d'un ou de plusieurs emplacements centraux et espacées autour d'une périphérie de la plateforme à une courte distance vers l'intérieur de la périphérie de la plateforme.

7. Dispositif selon une quelconque revendication précédente, dans lequel les mesures représentatives de la performance athlétique comportent une mesure représentative de la force cumulée sur une période de temps spécifiée.

8. Dispositif selon une quelconque revendication précédente, dans lequel les mesures représentatives de la performance athlétique comprennent une mesure représentative de la force d'impact moyenne sur une période de temps spécifiée.

9. Dispositif selon une quelconque revendication précédente, les données de la pluralité de cellules de charge sont enregistrées sous forme d'informations dans le domaine temporel représentatives de la force appliquée.

10. Dispositif selon la revendication 9 dans lequel les mesures sont dérivées des informations dans le domaine temporel, et dans lequel la catégorie prédéfinie d'exercice est un exercice réalisé de manière répétitive sous forme de répétitions, et dans lequel les informations dans le domaine temporel sont ainsi traitées pour identifier et évaluer des répétitions individuelles.

11. Dispositif selon une quelconque revendication précédente, dans lequel les éléments de connexion sont situés sous des ouvertures formées dans la surface supérieure.

12. Dispositif selon une quelconque revendication précédente, dans lequel chaque élément de connexion comporte une boucle, un loquet ou une barre cylindrique permettant la connexion des bandes de résistance.

13. Dispositif selon une quelconque revendication précédente, dans lequel les éléments de connexion sont disposés par paires opposées, permettant ainsi une bande de résistance pour chaque côté du corps.

14. Dispositif selon une quelconque revendication précédente, dans lequel les éléments de connexion comportent de multiples paires opposées des éléments de connexion.

15. Dispositif selon une quelconque revendication précédente comportant un ensemble de voyants lumineux (111) qui fournissent une sortie sous la forme d'indicateurs de force pour une quantité de force qu'un utilisateur applique à la plateforme.
